# EUROPÄISCHE PATENTSCHRIFT

(11) **EP 2 595 670 B1**
(45) Veröffentlichungstag und Bekanntmachung des Hinweises auf die Patenterteilung: **05.07.2017**
(21) Anmeldenummer: 11735798.8
(22) Anmeldetag: 18.07.2011
(51) Int. Cl.: A61M 1/14, F25B 21/04

(54) **ANORDNUNG ZUM ERWÄRMEN EINES MEDIZINISCHEN FLUIDS, MEDIZINISCHE FUNKTIONSEINRICHTUNG, MEDIZINISCHE BEHANDLUNGSVORRICHTUNG UND VERFAHREN**
ARRANGEMENT FOR HEATING A MEDICAL FLUID, MEDICAL FUNCTIONAL DEVICE, MEDICAL TREATMENT DEVICE, AND METHODS
SYSTÈME DE CHAUFFAGE D'UN FLUIDE MÉDICAL, APPAREIL MÉDICAL FONCTIONNEL, DISPOSITIF DE TRAITEMENT MÉDICAL ET PROCÉDÉS ASSOCIÉS

(30) Priorität: 20.07.2010 DE 102010031802
(43) Veröffentlichungstag der Anmeldung: 29.05.2013
(73) Patentinhaber: Fresenius Medical Care Deutschland GmbH, 61352 Bad Homburg (DE)
(72) Erfinder: PETERS, Arne, 61352 Bad Homburg (DE); HERRENBAUER, Michael, 61267 Neu-Anspach (DE); KREBER, Stefan, 66113 Saarbruecken (DE); WIKTOR, Christoph, 63571 Gelnhausen (DE)
(74) Vertreter: Bobbert & Partner Patentanwälte PartmbB
(86) Internationale Anmeldenummer: PCT/EP2011/003580
(87) Internationale Veröffentlichungsnummer: WO 2012/019693

(56) Entgegenhaltungen:
- WO-A1-03/055543
- FR-A1- 2 938 440
- US-A- 3 206 937
- US-A- 3 262 492
- US-A- 3 399 536
- US-A- 4 210 153
- US-A1- 2004 079 089

## Beschreibung

Die vorliegende Erfindung betrifft eine Anordnung gemäß Anspruch 1. Sie betrifft ferner eine medizinische Funktionseinrichtung gemäß Anspruch 14, eine medizinische Behandlungsvorrichtung gemäß Anspruch 15 sowie ein Verfahren gemäß Anspruch 16.

In der medizinischen Praxis ist es manchmal erforderlich, extrakorporal vorliegende physiologische Flüssigkeiten zu temperieren. So ist es zum Beispiel bei einer Dialyse üblich, Dialysierflüssigkeit und/oder Blut, besonders nach dessen Durchlauf durch den Dialysefilter, zu erwärmen. In Dokumenten US2004/079089, US 3262 492, WO 03/055543, US 3 399 536 und FR 2 938 440 sind einige Dialysesysteme beschrieben. Eine Aufgabe der vorliegenden Erfindung ist, eine weitere Anordnung zum Temperieren medizinischer Fluide vorzuschlagen. Ferner soll ein geeignetes Verfahren angegeben werden.

Diese Aufgabe wird durch eine Anordnung mit den Merkmalen des Anspruchs 1 gelöst. Sie wird ferner durch eine medizinische Funktionseinrichtung gemäß Anspruch 14, eine medizinische Behandlungsvorrichtung gemäße Anspruch 15 sowie ein Verfahren gemäß Anspruch 16 gelöst.

Alle oder manche mit der erfindungsgemäßen Anordnung erzielbaren Vorteile lassen sich ungeschmälert auch mit der medizinischen Funktionseinrichtung und/oder der medizinischen Behandlungsvorrichtung und/oder dem Verfahren erzielen.

Erfindungsgemäß wird eine Anordnung vorgeschlagen, welche zum Erwärmen eines medizinischen Fluids geeignet ist.

In manchen Ausführungsformen der Erfindung kann die erfindungsgemäße Anordnung zum Erwärmen des medizinischen Fluids vorgesehen und/oder eingesetzt sein.

Die erfindungsgemäße Anordnung weist wenigstens eine elektrisch betriebene erste Erwärmungseinrichtung auf. Die erste Erwärmungseinrichtung weist wenigstens einen sich bei deren Betrieb erwärmenden ersten Warmabschnitt und wenigstens einen sich bei deren Betrieb abkühlenden ersten Kaltabschnitt auf.

Ferner weist die erfindungsgemäße Anordnung einen ersten Bereich auf, welcher zum Aufnehmen des zu erwärmenden medizinischen Fluids geeignet und/oder vorgesehen ist. In bestimmten Ausführungsformen der vorliegenden Erfindung steht der erste Bereich in Wärmeaustauschbeziehung mit dem ersten Warmabschnitt der ersten Erwärmungseinrichtung. Ein zweiter Bereich der erfindungsgemäßen Anordnung steht in Wärmeaustauschbeziehung mit dem ersten Kaltabschnitt der Erwärmungseinrichtung.

In bestimmten Ausführungsformen der Erfindung ist genau das Gegenteil hiervon vorgesehen: Dort steht der erste Bereich in Wärmeaustauschbeziehung mit dem ersten Kaltabschnitt der ersten Erwärmungseinrichtung, und der zweite Bereich steht in Wärmeaustauschbeziehung mit dem ersten Warmabschnitt der ersten Erwärmungseinrichtung.

Die erfindungsgemäße medizinische Funktionseinrichtung weist wenigstens eine Anordnung gemäß der vorliegenden Erfindung auf.

Die erfindungsgemäße medizinische Behandlungsvorrichtung weist wenigstens eine Anordnung gemäß der vorliegenden Erfindung oder wenigstens eine medizinische Funktionseinrichtung gemäß der vorliegenden Erfindung auf.

Das erfindungsgemäße Verfahren dient dem Temperieren eines medizinischen Fluids und umfasst das Verwenden einer Anordnung gemäß der vorliegenden Erfindung.

Vorteilhafte Weiterbildungen der vorliegenden Erfindung sind jeweils Gegenstand der Unteransprüche.

Der Begriff "Warmabschnitt", wie er hierin verwendet wird, bezeichnet einen Abschnitt der ersten Erwärmungseinrichtung, welcher eine im Vergleich zum "Kaltabschnitt" der ersten Erwärmungseinrichtung höhere Temperatur aufweist; entsprechend weist der Kaltabschnitt im Vergleich zum Warmabschnitt eine niedrigere Temperatur auf.

Ein "Warmabschnitt", wie er hierin verwendet wird, bezeichnet in manchen erfindungsgemäßen Ausführungsformen einen Abschnitt der Erwärmungseinrichtung, welcher sich aufgrund des Zuführens elektrischer Energie erwärmt.

Ein "Kaltabschnitt", wie er hierin verwendet wird, bezeichnet in bestimmten erfindungsgemäßen Ausführungsformen einen Abschnitt der Erwärmungseinrichtung, welcher sich aufgrund des Zuführens elektrischer Energie abkühlt.

Unter einem "Abschnitt" kann erfindungsgemäß ein Teil einer Fläche oder die gesamte Fläche, zum Beispiel eine zur Wärmeaufnahme, Wärmeübertragung und/oder Wärmeabgabe zur Verfügung stehende Fläche, verstanden werden.

Insbesondere kann in manchen erfindungsgemäßen Ausführungsformen unter einem Warmabschnitt ein Abschnitt verstanden werden, der gezielt zum Erwärmen - oder zum vorübergehenden Abkühlen - eines Fluids vorgesehen ist.

Die Begriffe "Warmabschnitt" und "Kaltabschnitt" sind ausschließlich in Relation zueinander zu verstehen und es sollen hieraus in keiner Weise Rückschlüsse auf absolute Temperaturwerte des Warmabschnitts und des Kaltabschnitts gezogen werden.

Der Begriff "Wärmeaustauschbeziehung", wie er hierin verwendet wird, bezeichnet eine räumliche Beziehung zwischen zwei Strukturen, die einen Austausch oder einen Übergang von Wärme - oder auch Kälte - zwischen den zwei Strukturen erlaubt. Dabei kann der Übergang von Wärme auf jede bekannte Weise vorgesehen sein und/oder erfolgen, z.B. Konvektion, Wärmeübertragung, Wärmestrahlung.

Die "Wärmeaustauschbeziehung" geht in manchen erfindungsgemäßen Ausführungsformen mit einer entsprechenden körperlichen Ausgestaltung einher.

Der Begriff "Bereich", wie er hierin verwendet wird, bezeichnet allgemein einen Abschnitt, einen Raum, eine Fläche oder dergleichen, welche(r) eine Wärmeaustauschbeziehung mit entweder dem Warmabschnitt oder dem Kaltabschnitt einer Erwärmungseinrichtung eingehen kann oder welche(r) für einen Wärmeaustausch mit dem Warmabschnitt oder dem Kaltabschnitt der Erwärmungseinrichtung geeignet und vorgesehen ist.

In manchen Ausführungsformen der vorliegenden Erfindung kann der erste und/oder der zweite Bereich ein geschlossener Bereich wie z.B. ein Schlauchabschnitt, eine Leitung oder dergleichen sowie Abschnitte hiervon sein. In anderen Ausführungsformen kann der erste und/oder der zweite Bereich ein offener oder halboffener oder ein gelenkt oder ungelenkt durchströmter Bereich sein. Der erste und/oder der zweite Bereich kann ein begrenzter Bereich sein. Der erste und/oder der zweite Bereich kann ein Bereich in mittelbarer oder unmittelbarer Umgebung bzw. Nähe der erfindungsgemäßen Anordnung sein.

In bestimmten Ausführungsformen der vorliegenden Erfindung ist der zweite Bereich ein Festkörper, wie ein Kühlkörper oder Kühlblock, eine Kühlrippe, eine Wärmebrücke oder dergleichen sein.

In bestimmten Ausführungsformen der vorliegenden Erfindung ist der zweite Bereich vorgesehen zum Aufnehmen eines zweiten Fluids.

In manchen Ausführungsformen der vorliegenden Erfindung ist der erste Bereich Teil eines Fluidkreislaufs. Der erste Bereich ist in bestimmten Ausführungsformen ein Fluidbereich.

In bestimmten Ausführungsformen der vorliegenden Erfindung ist der zweite Bereich Teil eines Fluidkreislaufs. Der zweite Bereich ist in bestimmten Ausführungsformen ein Fluidbereich.

In bestimmten Ausführungsformen der vorliegenden Erfindung sind der erste Bereich und der zweite Bereich Teil - desselben, in manchen Ausführungsformen jeweils Teil verschiedener - Fluidkreisläufe.

Dabei ist ein Fluidkreislauf in manchen erfindungsgemäßen Ausführungsformen eine geschlossene Führung des Fluids. In bestimmten erfindungsgemäßen Ausführungsformen ist der Fluidkreislauf hingegen nicht geschlossen; z.B. fließt durch den Fluidkreislauf in solchen Ausführungsformen Fluid von einer Quelle um nach seiner Verwendung verworfen zu werden ohne zur Quelle zurückgeführt zu werden. Auch für derartige Ausgestaltungen wird hier der Begriff "Kreislauf" verwendet.

In bestimmten Ausführungsformen der vorliegenden Erfindung ist der erste Bereich Teil eines Fluidkreislaufs, der zweite Bereich ist kein Teil eines Fluidkreislaufs oder umgekehrt.

In bestimmten Ausführungsformen der vorliegenden Erfindung sind der erste Bereich und/oder der zweite Bereich zum gezielten Führen oder Leiten eines Fluids ausgestaltet.

In manchen Ausführungsformen der Erfindung kann der Bereich eine Leitung sein. Das Fluid kann in der Leitung geführt oder geleitet werden.

Die Leitung kann eine definierte Leitung sein. Die Leitung kann Bestandteil der erfindungsgemäßen Anordnung sein.

Die Leitung kann eine - im Bezug auf die erfindungsgemäße Anordnung - externe Einrichtung sein. Die Leitung kann mit der erfindungsgemäßen Anordnung verbindbar oder verbunden sein. Die Leitung kann Teil eines (ggf. übergeordneten) Fluidkreislaufs sein.

Der Begriff "Erwärmungseinrichtung", wie er hierin verwendet wird, bezeichnet eine Einrichtung, welche geeignet und/oder vorgesehen ist, ein medizinisches Fluid zu Erwärmen.

In bestimmten Ausführungsformen ist die erfindungsgemäße Anordnung zum Abkühlen des medizinischen Fluids geeignet und/oder vorgesehen.

In bestimmten Ausführungsformen der vorliegenden Erfindung ist die erfindungsgemäße Anordnung vorgesehen zum Erwärmen des medizinischen Fluids in einem ersten Zeitabschnitt und vorgesehen zum Abkühlen des medizinischen Fluids in einem zweiten Zeitabschnitt, welcher vom ersten Zeitabschnitt verschieden ist.

In manchen Ausführungsformen weist die erfindungsgemäße Anordnung eine Einrichtung auf, welche geeignet und/oder vorgesehen ist zum zumindest vorübergehenden Veranlassen einer Abkühlung des medizinischen Fluids mittels des ersten Warmabschnitts, anstelle dieses mittels des Warmabschnitts zu erwärmen. Dies kann z.B. durch Umkehr einer Stromzufuhr für die elektrisch betriebene erste Erwärmungseinrichtung eingeleitet werden.

Eine solche Einrichtung kann ein Schalter sein, z.B. ein Schalter bzw. Umschalter in einem Stromkreis, in welchen die erste Erwärmungseinrichtung zum Zwecke ihrer Stromzufuhr integriert ist.

Die erste Erwärmungseinrichtung kann ein Thermoelement sein oder ein solches Thermoelement aufweisen.

In bestimmten Ausführungsformen der vorliegenden Erfindung ist die erste Erwärmungseinrichtung ein Peltier-Element oder weist ein solches auf. In manchen Ausführungsformen der vorliegenden Erfindung weist die erste Erwärmungseinrichtung eine Mehrzahl von Peltier-Elementen auf.

In bestimmten Ausführungsformen der vorliegenden Erfindung weist die Anordnung eine elektrisch betriebene zweite Erwärmungseinrichtung mit wenigstens einem sich bei deren Betrieb erwärmenden zweiten Warmabschnitt und wenigstens einem sich bei deren Betrieb abkühlenden zweiten Kaltabschnitt auf.

Dabei sind Erwärmung und Abkühlung in manchen erfindungsgemäßen Ausführungsformen jeweils durch die Inbetriebnahme oder die Stromzufuhr bewirkt oder veranlasst, in bestimmten erfindungsgemäßen Ausführungsformen aber zumindest auf diese zurückzuführen.

Die zweite Erwärmungseinrichtung ist in bestimmten Ausführungsformen dazu geeignet und/oder vorgesehen, ein Fluid, insbesondere das medizinische Fluid, zu erwärmen oder zu dessen Erwärmung beizutragen.

Alle vorstehend in Verbindung mit der ersten Erwärmungseinrichtung beschriebenen Ausführungen können in bestimmten Ausführungsformen auch auf die zweite Erwärmungseinrichtung zutreffen und umgekehrt.

In Ausführungsformen, in denen die erfindungsgemäße Anordnung zwei Erwärmungseinrichtungen aufweist, kann der mit dem ersten Bereich in Wärmeaustauschbeziehung stehende Warmabschnitt der erste Warmabschnitt der ersten Erwärmungseinrichtung sein. Der mit dem zweiten Bereich in Wärmeaustauschbeziehung stehende Kaltabschnitt kann der zweite Kaltabschnitt der zweiten Erwärmungseinrichtung sein. In anderen Ausführungsformen der vorliegenden Erfindung kann dies genau umgekehrt sein.

In bestimmten Ausführungsformen der vorliegenden Erfindung ist der zweite Bereich mit dem ersten Kaltabschnitt der ersten Erwärmungseinrichtung zum Erwärmen des ersten Kaltabschnitts oder des zweiten Kaltabschnitts verbindbar oder verbunden. In manchen Ausführungsformen der vorliegenden Erfindung ist der zweite Bereich mit dem zweiten Kaltabschnitt der zweiten Erwärmungseinrichtung verbindbar oder verbunden.

Die Wärmeaustauschbeziehung kann erfindungsgemäß eine Beziehung oder eine Verbindung sein. Die Verbindung kann eine physische Verbindung sein. In bestimmten Ausführungsformen der vorliegenden Erfindung steht der zweite Bereich in physischem Kontakt mit dem zu erwärmenden Kaltabschnitt.

Ein Erwärmen des ersten Kaltabschnitts oder des zweiten Kaltabschnitts kann insbesondere durch Übertragen von Wärme aus dem zweiten Fluid des zweiten Bereichs auf den entsprechenden Kaltabschnitt erreicht werden.

Das zweite Fluid ist eine Flüssigkeit oder ein Gas, welche(s) vorgesehen ist, Wärme (aus dem oder mittels des zweiten Bereichs) an einen Kaltabschnitt (den ersten Kaltabschnitt der ersten Erwärmungseinrichtung oder den zweiten Kaltabschnitt der zweiten Erwärmungseinrichtung) abzugeben oder zu übertragen.

In bestimmten Ausführungsformen der vorliegenden Erfindung sind das medizinische Fluid und das zweite Fluid im Wesentlichen gleich.

Das medizinische Fluid kann eine medizinische Flüssigkeit wie Blut, insbesondere eine extrakorporale Flüssigkeit wie Blut, Dialysierflüssigkeit, Substituatflüssigkeit, Medikamentenlösungen und dergleichen, Kombinationen oder Mischungen derselben sein.

In bestimmten Ausführungsformen der vorliegenden Erfindung ist das medizinische Fluid Dialysierflüssigkeit oder extrakorporales Blut.

In bestimmten Ausführungsformen der vorliegenden Erfindung weist die erfindungsgemäße Anordnung wenigstens einen Wärmeüberträgerfluidkreislauf mit einem ersten und einem zweiten Wärmeüberträgerbereich auf.

Der Wärmeüberträgerfluidkreislauf ist vorzugsweise ein geschlossener Fluidkreislauf, z.B. ein in sich geschlossener Fluidkreislauf.

Der erste Wärmeüberträgerbereich und der zweite Wärmeüberträgerbereich sind dazu vorgesehen, ein Wärmeüberträgerfluid aufzunehmen.

Der Begriff "Wärmeüberträgerfluid", wie er hierin verwendet wird, bezeichnet ein Fluid, welches zum Übertragen von Wärme geeignet ist. Das Wärmeüberträgerfluid kann zum Übertragen von Wärme vorgesehen sein.

Beispiele für geeignete Wärmeüberträgerfluide schließen Wasser, Öl und dergleichen ein.

Im Wärmeüberträgerfluidkreislauf kann das Wärmeüberträgerfluid selbsttätig zirkulieren oder unterstützend oder ausschließlich durch (wenigstens eine) geeignete und dazu vorgesehene Einrichtung, wie eine Fördereinrichtung, gefördert werden.

In bestimmten Ausführungsformen der erfindungsgemäßen Anordnung ist der Wärmeüberträgerfluidkreislauf zwischen dem ersten Bereich und dem zweiten Bereich angeordnet.

Der Wärmeüberträgerfluidkreislauf kann derart zwischen dem ersten Bereich und dem zweiten Bereich angeordnet sein, dass der erste Warmabschnitt der ersten Erwärmungseinrichtung in Wärmeaustauschbeziehung mit dem ersten Bereich steht; der erste Kaltabschnitt der ersten Erwärmungseinrichtung steht in Wärmeaustauschbeziehung mit dem ersten Wärmeüberträgerbereich; der zweite Warmabschnitt der zweiten Erwärmungseinrichtung in Wärmeaustauschbeziehung steht mit dem zweiten Wärmeüberträgerbereich; und der zweite Kaltabschnitt der zweiten Erwärmungseinrichtung steht in Wärmeaustauschbeziehung mit dem zweiten Bereich.

Das Wärmeüberträgerfluid kann in solchen Ausführungsformen als Mittel zum Übertragen von Wärme (vom zweiten Bereich über den Wärmeüberträgerfluidkreislauf) auf den ersten Kaltabschnitt einsetzbar sein.

Die Verwendung eines Wärmerüberträgerfluidkreislaufs zwischen dem ersten und dem zweiten Bereich kann insbesondere von Vorteil sein, wenn das medizinische Fluid und das zweite Fluid hierdurch physikalisch bzw. räumlich voneinander getrennt werden sollen. Dies schließt eine Kontaminations- oder Verunreinigungsgefahr für das medizinische Fluid durch das zweite Fluid aus.

In bestimmten Ausführungsformen der vorliegenden Erfindung ist die erfindungsgemäße Anordnung als wiederverwendbare Einheit ausgestaltet. In solchen Ausführungsformen ist die erfindungsgemäße Anordnung zu einer mehrmaligen Verwendung bzw. zu einem mehrmaligen Gebrauch vorgesehen.

Die erfindungsgemäße Anordnung kann fest installierte Leitungen, vor allem mehrfach zu benutzende, oder nicht fest installierte Leitungen aufweisen oder mit fest installierten Leitungen oder mit nicht fest installierten Leitungen verbunden sein. In bestimmten Ausführungsformen kann die erfindungsgemäße Anordnung mit Leitungen verbunden sein, welche fest an einer medizinischen Behandlungsvorrichtung installiert sind.

In bestimmten anderen Ausführungsformen der vorliegenden Erfindung ist die erfindungsgemäße Anordnung als Einmalteil oder Disposable oder Wegwerfartikel ausgestaltet. In solchen Ausführungsformen ist vorgesehen, die erfindungsgemäße Anordnung nur einmal zu gebrauchen und nach ihrem einmaligen Gebrauch zu entsorgen.

Die erfindungsgemäße Anordnung kann als Teil einer medizinischen Funktionseinrichtung ausgestaltet sein.

In manchen Ausführungsformen ist die erfindungsgemäße Anordnung integral mit einer medizinischen Funktionseinrichtung ausgestaltet bzw. in eine medizinische Funktionseinrichtung integriert.

In bestimmten Ausführungsformen ist die medizinische Funktionseinrichtung als Einmalteil oder Disposable oder Wegwerfartikel ausgestaltet. In bestimmten Ausführungsformen ist die medizinische Funktionseinrichtung eine (Disposable-)Blutkassette.

Die medizinische Funktionseinrichtung kann eine Kassette nach Art einer sleepsafe-Kassette, hergestellt von Fresenius Medical Care (Bad Homburg, Deutschland), sein.

Der *sleep safe PD cycler* ist ein automatisierter Peritoneal-Cycler für den Haus- und Klinikgebrauch. Die Pumpwirkung wird durch eine hydraulisch betriebene Membrankolbenpumpe sichergestellt.

Das *sleep safe* set ist ein Einmalartikel mit halbrunden Kammern, in denen das hydraulische Medium entweder die Lösung durch eine Membran aus der Kammer herausdrückt oder die Lösung in die Kammer hineinzieht. Eine integrierte Heizeinrichtung erwärmt die Dialyselösung vor ihrer Infusion in den Patienten. Ein automatisches Konnektionssystem verbindet die Lösungsbeutel mit dem Schlauchset. Verschiedene Drucksensoren überwachen die Drücke im System. Das System wird von einem Mikroprozessor gesteuert und überwacht. Ein Prozessor stellt ein unabhängiges Sicherheitssystem dar.

Eine ausführliche Beschreibung einer *sleepsafe*-Kassette kann der Patentschrift Nr. EP 0 956 876 B1 entnommen werden, auf deren diesbezügliche Offenbarung hiermit vollinhaltlich Bezug genommen wird.

Die erfindungsgemäße medizinische Funktionseinrichtung kann wenigstens einen Fluidkreislauf, zum Beispiel zwei Fluidkreisläufe, zum Beispiel für (frische und gebrauchte) Dialysierflüssigkeit oder für extrakorporales Blut (vor und nach einer Behandlungseinrichtung, wie einem Blutbehandlungsfilter) aufweisen.

Die erfindungsgemäße Anordnung kann funktionell an eine medizinische Behandlungsvorrichtung koppelbar oder gekoppelt sein.

Geeignete medizinische Behandlungsvorrichtungen können Blutbehandlungsvorrichtungen, insbesondere Dialysiervorrichtungen, wie eine Akut-Dialysiervorrichtung, und dergleichen einschließen.

Das erfindungsgemäße Verfahren kann zum Erwärmen eines medizinischen Fluids eingesetzt werden.

Dies kann in bestimmten Ausführungsformen des erfindungsgemäßen Verfahrens beinhalten - unter Verwenden der erfindungsgemäßen Anordnung -, zunächst ein medizinisches Fluids in den ersten Bereich einzubringen.

Das medizinische Fluid wird im ersten Bereich mittels der ersten Erwärmungseinrichtung erwärmt durch Übertragen von Wärme vom ersten Warmabschnitt der ersten Erwärmungseinrichtung auf den ersten Bereich.

In bestimmten Ausführungsformen der vorliegenden Erfindung ist vorgesehen, den ersten Kaltabschnitt der ersten Erwärmungseinrichtung oder den zweiten Kaltabschnitt der zweiten Erwärmungseinrichtung durch Übertragen von Wärme des zweiten Fluids des zweiten Bereichs auf den ersten Kaltabschnitt der ersten Erwärmungseinrichtung oder den zweiten Kaltabschnitt der zweiten Erwärmungseinrichtung zu erwärmen.

In anderen Ausführungsformen des erfindungsgemäßen Verfahrens wird insbesondere eine Anordnung gemäß der vorliegenden Erfindung mit zwei Erwärmungseinrichtungen und einem Wärmeüberträgerfluidkreislauf eingesetzt.

Der Wärmeüberträgerfluidkreislauf kann, wie vorstehend diesbezüglich ausgeführt ist, ausgestaltet und/oder angeordnet sein.

In solchen Ausführungsformen des erfindungsgemäßen Verfahrens kann das Verfahren das Erwärmen des zweiten Kaltabschnitts der zweiten Erwärmungseinrichtung durch Übertragen von Wärme des zweiten Fluids des zweiten Bereichs hierauf umfassen. Es kann ferner das Erwärmen des Wärmeüberträgerfluids im zweiten Wärmeüberträgerbereich mittels der zweiten Erwärmungseinrichtung durch Übertragen von Wärme vom zweiten Warmabschnitt der zweiten Erwärmungseinrichtung auf den zweiten Wärmeüberträgerbereich umfassen. Es kann zudem das Übertragen von Wärme des Wärmeüberträgerfluids des ersten Wärmeüberträgerbereichs auf den ersten Kaltabschnitt der ersten Erwärmungseinrichtung umfassen.

Ein Austausch von Wärme oder ein Übertragen von Wärme des Wärmeüberträgerfluids vom zweiten Wärmeüberträgerbereich auf den ersten Wärmeüberträgerbereich kann durch Leiten oder Führen des Wärmeüberträgerfluids innerhalb eines Inneren des Wärmeüberträgerfluidkreislaufs erreicht werden.

Dabei kann sich das Wärmeüberträgerfluid in manchen Ausführungsformen der vorliegenden Erfindung selbsttätig durch ein Inneres des Wärmeüberträgerfluidkreislaufs bewegen. In manchen Ausführungsformen kann hierzu eine geeignete Einrichtung, wie eine Fördereinrichtung zum Fördern des Wärmeüberträgerfluids innerhalb des Wärmeüberträgerfluidkreislaufs, eingesetzt werden.

In bestimmten Ausführungsformen dient das erfindungsgemäße Verfahren zum Erwärmen eines medizinischen Fluids in einem ersten Zeitabschnitt und zum Abkühlen des medizinischen Fluids in einem zweiten Zeitabschnitt, welcher vom ersten Zeitabschnitt verschieden ist.

In solchen Ausführungsformen des erfindungsgemäßen Verfahrens kann vorgesehen sein, das medizinische Fluid im ersten Bereich mittels der ersten Erwärmungseinrichtung im ersten Zeitabschnitt zu erwärmen. Alternativ hierzu kann, z.B. durch Verändern der Stromzufuhr (oder Umkehr der Stromflussrichtung) für die elektrisch betriebene erste und/oder die zweite Erwärmungseinrichtung, bewirkt werden, dass das medizinische Fluid im ersten Bereich mittels des ersten Warmabschnitts der ersten Erwärmungseinrichtung nicht erwärmt, sondern durch Übertragen von Kälte vom ersten Warmabschnitt der ersten Erwärmungseinrichtung abgekühlt wird.

Die Veränderung der Stromzufuhr kann in manchen Ausführungsformen unter Verwenden eines Schalters, vorgesehen in einem Stromkreis zur Versorgung/Stromzufuhr der ersten Erwärmungseinrichtung und/oder der zweiten Erwärmungseinrichtung, erreicht werden.

Ausführungsformen der vorliegenden Erfindung können einen oder mehrere der im Folgenden beschriebenen Vorteile aufweisen.

Die vorliegende Erfindung stellt eine Anordnung bereit, welche in einfacher und technisch wenig aufwendiger Weise erlaubt, ein zweites Fluid, zum Beispiel insbesondere Abwärme eines Behandlungssystems wie beispielsweise von Aktoren, Sensoren und/oder Elektronik, zur Erwärmung von bestimmten Abschnitten von Erwärmungseinrichtungen wie z.B. Kaltabschnitten von Peltier-Elementen oder dergleichen während des Erwärmens eines medizinischen Fluids einzusetzen.

Auf diese Weise kann es in bestimmten Ausführungsformen der vorliegenden Erfindung vorteilhaft möglich sein, auf Heizungen zur Blut- und/oder Dialysaterwärmung zu verzichten und damit vorteilhaft einen größeren Energiebedarf der Dialysemaschinen zu vermeiden.

Dies kann insbesondere vorteilhaft dazu beitragen, höhere Temperaturen im Maschineninnenraum zu vermeiden.

Durch die Nutzung von Abwärme kann in bestimmten Ausführungsformen der vorliegenden Erfindung vorteilhaft eine hohe Energieeffizienz des Systems erreicht werden.
So kann das zweite Fluid, welches zum Erwärmen des Kaltabschnitts der Erwärmungseinrichtung eingesetzt wird, in bestimmten Ausführungsformen der vorliegenden Erfindung Abwärme elektronischer Bauteile, z.B. die in Dialysemaschinen vorhandene Abwärme von Geräteteilen sowie die Abwärme von verbrauchtem Dialysat, führen.

Durch einen aktiven Wärmeaustausch zwischen frischem und gebrauchtem Dialysat kann die Energieeffizienz des Systems vorteilhaft weiter gesteigert werden.

Eine Nutzung solcher Abwärme kann in bestimmten Ausführungsformen vorteilhaft dazu beitragen, ein ungenutztes Verwerfen oder gar ein - unter Aufbringen von zusätzlicher Energie - erforderliches Abführen der Abwärme (z.B. unter Einsatz von Lüftern, Ventilatoren, entsprechenden Baumaßnahmen und dergleichen) zu reduzieren oder sogar zu verhindern.

Daneben kann es in bestimmten Ausführungsformen der vorliegenden Erfindung vorteilhaft möglich sein, auf große Wärmeaustauschflächen an verschiedenen Komponenten zu verzichten.

Ferner kann es in bestimmten Ausführungsformen der vorliegenden Erfindung vorteilhaft möglich sein, eine kompaktere und einfachere Bauweise der Maschine durch reduzierte Wärmeaustauschflächen bereitzustellen.

Durch Verzicht auf (einen) Lüfter oder entsprechend groß dimensionierte Lüfter kann es vorteilhaft möglich sein, die Lautstärke der Anordnung deutlich zu verringern. Der Verzicht auf (einen) Lüfter, welcher i.d.R. einen hohen Volumenstrom und eine kurze Lebenszeit aufweist, kann zudem vorteilhaft zu einer Einsparung des Kosten- und/oder Wartungsaufwands der erfindungsgemäßen Anordnung beitragen.

Bei bestimmten erfindungsgemäßen Anordnungen kann die Menge an elektrischer Energie im Vergleich zu zum Beispiel einer klassischen Widerstandsheizung vorteilhaft reduziert sein.

Ferner kann es mit der erfindungsgemäßen Anordnung in bestimmten Ausführungsformen der vorliegenden Erfindung möglich sein, die zum Beispiel in einer gebrauchten Dialysierflüssigkeit vorliegende Wärmemenge auf vorteilhaft einfache Weise nutzbringend einzusetzen. So kann in manchen erfindungsgemäßen Ausführungsformen ein aufwendiges Rückgewinnen durch raumfordernde passive Wärmetauscher oder gar ein Verlust durch Verwerfen der Dialysierflüssigkeit vermieden werden. Insbesondere der hierbei mögliche Gewinn an Bauraum, aber auch weitere Vorteile, können bei Akutdialysesystemen von Vorteil sein.

Durch Zufuhr bzw. Übertragung von Wärme bzw. Wärmeenergie auf den Kaltabschnitt einer Erwärmungseinrichtung kann es in bestimmten Ausführungsformen der vorliegenden Erfindung vorteilhaft möglich sein, eine Temperaturdifferenz zwischen dem Kaltabschnitt und dem Warmabschnitt der Erwärmungseinrichtung zu verringern. Dadurch kann es vorteilhaft möglich sein, eine Verlustwärme eines Behandlungssystems zu reduzieren.

Daneben kann es vorteilhaft möglich sein, insgesamt niedrigere Temperaturen in der Maschine zu erreichen.

Mittels der erfindungsgemäßen Anordnung kann ein Temperaturunterschied zwischen dem Kaltabschnitt und dem Warmabschnitt der Erwärmungseinrichtung in bestimmten Ausführungsformen der vorliegenden Erfindung vorteilhaft derart verringert werden, dass ein Wärmeverlust bzw. Verlust an Wärmeenergie durch Wärmefluss vom Warmabschnitt zum Kaltabschnitt, welcher sich insbesondere bei relativ hohen Temperaturunterschieden einstellen kann, vorteilhaft vermieden werden kann.

Ein Wirkungsgrad der erfindungsgemäßen Anordnung und/oder eines Behandlungssystems kann somit verbessert oder sogar optimiert sein. Daneben kann es vorteilhaft möglich sein, den Stromverbrauch zu senken.

Dies kann insbesondere in Behandlungssystemen von Vorteil sein, in denen große Mengen an physiologischen Flüssigkeiten zu erwärmen sind. Beispiele schließen Blutbehandlungssysteme wie Dialysiervorrichtungen und dergleichen ein, bei denen Flussraten von 500 ml/min (oder mehr) des Dialysierflüssigkeitskreislaufs auftreten können.

Mittels der vorliegenden Erfindung kann es vorteilhaft möglich sein, auch größere oder große Mengen der dem Patienten zu infundierenden medizinischen Fluide ausreichend zu erwärmen und so ein Auskühlen des Patienten vorteilhaft zu vermeiden.

Durch Verwendung eines oder mehrerer Peltier-Elemente oder bestimmter anderer Elemente, bei denen Strom zur Erzeugung thermischer Energie eingesetzt wird, kann es in bestimmten Ausführungsformen der vorliegenden Erfindung vorteilhaft auf einfache und wenig aufwendige Weise möglich sein, durch eine bloße Stromflussrichtungsumkehr zwischen einem Erwärmen und einem Abkühlen des zu temperierenden medizinischen Fluids auszuwählen, abzuwechseln, umzukehren oder umzuschalten. Hierdurch kann es in manchen Ausführungsformen der vorliegenden Erfindung vorteilhaft möglich sein, zum Beispiel kurzfristig Energie aus einem Dialysierflüssigkeitskreislauf zu entziehen, und so vorteilhaft z.B. im Falle eines Stopps des Dialysierflüssigkeitskreislaufs ein Überhitzen der Flüssigkeit und/oder der Erwärmungseinrichtung sowie ein Überhitzen an den Schnittstellen zu verhindern. Dies kann in Momenten des vorübergehenden Stillstandes des Fluids, z.B. der Dialysierflüssigkeit oder des Bluts, im ersten Bereich, in welcher keine Erwärmung erforderlich ist und eine Überhitzung drohen kann, von besonderem Vorteil sein. Entsprechende Temperatursensoren, - fühler und/oder -regler können erfindungsgemäß vorgesehen sein.

Aufgrund ihrer kompakten Bauweise und/oder ihrer kompakten Abmessungen können Peltier-Elemente vorteilhaft in Bereichen, bei denen nur ein geringes Raumangebot vorhanden ist, zum Beispiel auf einer Blutkassette zur Dialyse, verwendet werden.

Dies kann insbesondere im Infusionsbereich und/oder im Dialysebereich von Vorteil sein, bei denen Anforderungen an die Kompaktheit von Therapiegeräten gestellt werden. Zudem kann es vorteilhaft möglich sein, fluidführende Einmalartikel kompakt zu gestalten. Auf diese Weise kann vorteilhaft eine immer größere Anzahl von Funktionen auf einem Einmalartikel realisiert werden.

Die erfindungsgemäße Anordnung kann zum Erwärmen von Dialysierflüssigkeit im Rahmen einer extrakorporalen Blutbehandlung eingesetzt werden. Eine solche beispielhafte Verwendung der erfindungsgemäßen Anordnung ist anhand der beigefügten Zeichnung beschrieben. Die vorliegende Erfindung ist jedoch nicht hierauf beschränkt.

Im Folgenden wird die vorliegende Erfindung beispielhaft unter Bezugnahme auf die beigefügte Zeichnung beschrieben. In der Zeichnung bezeichnen identische Bezugszeichen gleiche oder identische Elemente. Es gilt:
- Fig. 1: zeigt schematisch vereinfacht eine erfindungsgemäße Anordnung gemäß einer ersten Ausführungsform;
- Fig. 2: zeigt schematisch vereinfacht eine erfindungsgemäße Anordnung gemäß einer zweiten Ausführungsform;
- Fig. 3: zeigt schematisch vereinfacht eine erfindungsgemäße Anordnung gemäß einer dritten Ausführungsform;
- Fig. 4A: zeigt schematisch vereinfacht eine erfindungsgemäße Anordnung gemäß einer vierten Ausführungsform in perspektivischer Ansicht;
- Fig. 4B: zeigt schematisch vereinfacht die erfindungsgemäße Anordnung an einer Funktionseinrichtung;
- Fig. 5: zeigt schematisch vereinfacht eine erfindungsgemäße Anordnung gemäß einer fünften Ausführungsform;
- Fig. 6: zeigt schematisch vereinfacht ein Wärmemanagement mittels der erfindungsgemäßen Anordnung;
- Fig. 7: zeigt ein weiteres Wärmemanagement mittels der erfindungsgemäßen Anordnung;
- Fig. 8: zeigt ein weiteres Wärmemanagement mittels der erfindungsgemäßen Anordnung; und
- Fig. 9: zeigt schematisch vereinfacht eine erfindungsgemäße Anordnung an einer Behandlungsvorrichtung.

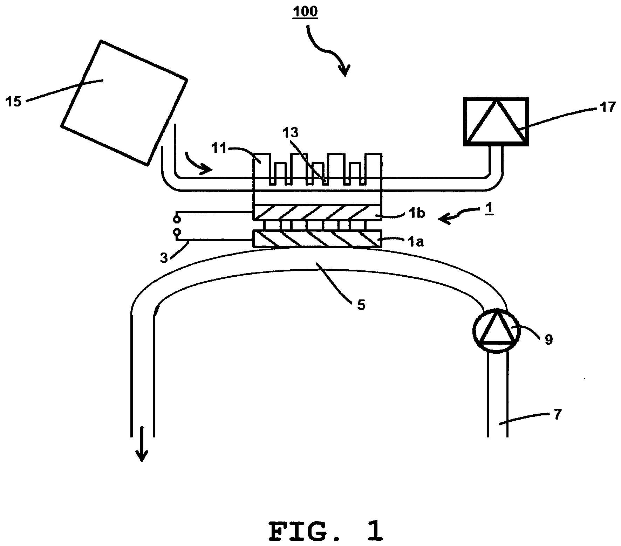

**Fig. 1** zeigt schematisch vereinfacht eine erfindungsgemäße Anordnung 100 in einer ersten Ausführungsform.

Die erfindungsgemäße Anordnung 100 weist eine erste Erwärmungseinrichtung 1 auf. Die erste Erwärmungseinrichtung 1 kann ein Peltier-Element sein oder ein solches aufweisen.

Die erste Erwärmungseinrichtung 1 weist einen sich bei deren Betrieb erwärmenden ersten Warmabschnitt 1a und einen sich bei deren Betrieb abkühlenden ersten Kaltabschnitt 1b auf.

Zum Betreiben der ersten Erwärmungseinrichtung 1 ist diese in einen Stromkreis 3 integriert.

Die erfindungsgemäße Anordnung 100 weist einen ersten Bereich 5 auf. Der erste Bereich 5 steht in Wärmeaustauschbeziehung mit dem ersten Warmabschnitt 1a der ersten Erwärmungseinrichtung 1.

Der erste Bereich 5 ist in Fig. 1 vorgesehen, Dialysierflüssigkeit bzw. Dialysat, genauer gesagt frische Dialysierflüssigkeit, aufzunehmen.

Die frische Dialysierflüssigkeit kann in einer Dialysierflüssigkeitsleitung 7 vorliegen, welche den ersten Bereich 5 aufweist, wie dies in Fig. 1 beispielhaft veranschaulicht ist. Die Dialysierflüssigkeitsleitung 7 kann Teil eines Dialysierflüssigkeitskreislaufs - geschlossen oder (halb-)offen, zum Beispiel als Teil eines übergeordneten Dialysierflüssigkeitskreislaufs - sein.

Innerhalb der Dialysierflüssigkeitsleitung 7 ist eine Fördereinrichtung 9 zum Fördern der Dialysierflüssigkeit in einem Inneren der Dialysierflüssigkeitsleitung 7 vorgesehen.

Die Dialysierflüssigkeit kann in Richtung einer medizinischen Behandlungseinrichtung (in Fig. 1 nicht gezeigt) gefördert werden, wie dies anhand des (in der Darstellung der Fig. 1) nach unten gerichteten Pfeils angedeutet ist. Die medizinische Behandlungseinrichtung kann ein Dialysefilter z.B. eines Akutdialysesystems sein.

Am Kaltabschnitt 1b der ersten Erwärmungseinrichtung 1 ist, wie in Fig. 1 gezeigt, optional ein Kühlkörper 11 vorgesehen. Ein solcher Kühlkörper 11 muss jedoch nicht zwingend vorgesehen sein.

Der Kühlkörper 11 kann in oder an einem zweiten Bereich 13 der ersten Erwärmungseinrichtung 1 angeordnet sein wie in Fig. 1 exemplarisch gezeigt. Der Kühlkörper 11 kann eine Kühlwirkung des zweiten Bereichs 13 verstärken.

Ein zweiter Bereich 13 weist ein zweites Fluid, wie in Fig. 1 beispielhaft gezeigt, zum Beispiel Luft, auf. Die Luft kann durch Abwärme aus elektronischen Bauteilen (zusammengefasst unter dem Bezugszeichen 15) einer medizinischen Behandlungseinrichtung erwärmt sein.

Das Leiten oder Führen des zweiten Fluids ist in Fig. 1 anhand der gebogenen Struktur verdeutlicht. Die gebogene Struktur muss keine Leitung sein. Sie muss generell kein (ab)geschlossener Raum/Bereich sein. Ein Lüfter 17 kann zum Leiten des zweiten Fluids zum ersten Kaltabschnitt 1b eingesetzt werden. Eine Strömungsrichtung des zweiten Fluids ist beispielhaft anhand des gebogenen Pfeils gezeigt.

Der zweite Bereich 13 kann vielmehr ein im Wesentlichen unbegrenzter Bereich sein, z.B. die Atmosphäre (zumindest derjenige Bereich in unmittelbarer Umgebung des ersten Kaltabschnitts 1b).

Das zweite Fluid kann Wärmeenergie an bzw. auf den ersten Kaltabschnitt 1b der ersten Erwärmungseinrichtung 1 übertragen.

Ggf. nach Passieren des zweiten Bereichs 13 noch vorhandene Restwärme des zweiten Fluids kann mit Hilfe des Lüfters 17 entzogen werden.

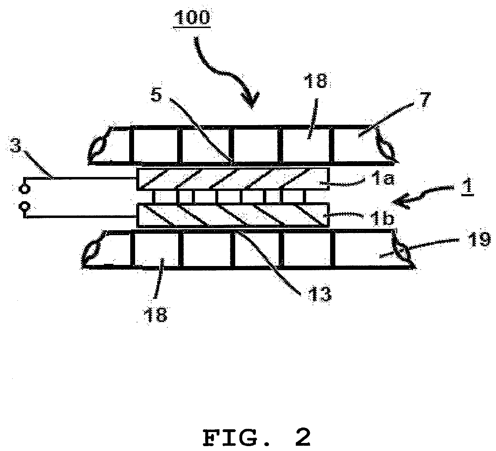

**Fig. 2** zeigt schematisch vereinfacht eine erfindungsgemäße Anordnung 100 in einer zweiten Ausführungsform.

Die erfindungsgemäße Anordnung 100 kann Teil einer medizinischen Funktionseinrichtung sein.

Eine solche medizinische Funktionseinrichtung kann dabei eine oder mehrere erfindungsgemäße Anordnungen 100 aufweisen. Die medizinische Funktionseinrichtung kann wenigstens zwei Fluidkreisläufe oder Abschnitte hiervon aufweisen, zum Beispiel einen für frische und einen für gebrauchte Dialysierflüssigkeit.

Geeignete Fluidkreisläufe können mäanderförmig auf bzw. in der medizinischen Funktionseinrichtung angeordnet sein. Eine beispielhafte Mäanderstruktur 18 ist gezeigt.

Eine geeignete medizinische Funktionseinrichtung kann eine Kassette, insbesondere eine Blutkassette sein.

Wie in Fig. 2 gezeigt, ist eine Dialysierflüssigkeitsleitung 19 für gebrauchte Dialysierflüssigkeit derart angeordnet, dass ein zweiter Bereich 13 der Dialysierflüssigkeitsleitung 19 in geeigneter Weise mit dem ersten Kaltabschnitt 1b der ersten Erwärmungseinrichtung 1 in Wärmeaustauschbeziehung steht.

Das zweite Fluid ist im Beispiel der Fig. 2 im Wesentlichen oder ausschließlich gebrauchtes Dialysat.

Die Dialysierflüssigkeitsleitung 7 enthält im Wesentlichen frische Dialysierflüssigkeit.

Die Dialysierflüssigkeitsleitung 7 weist den ersten Bereich 5 auf, der in geeigneter Weise mit dem ersten Warmabschnitt 1a der ersten Erwärmungseinrichtung 1 in Wärmeaustauschbeziehung steht.

Während des Betriebs der ersten Erwärmungseinrichtung 1 wird durch Zufuhr elektrischer Energie mittels des Stromkreises 3 Wärme vom Kaltabschnitt 1b auf den Warmabschnitt "gepumpt" bzw. ganz allgemein gesprochen, übertragen.

Gleichzeitig wird der gebrauchten Dialysierflüssigkeit der Dialysierflüssigkeitsleitung 19 Wärme entzogen und zum Erwärmen des ersten Kaltabschnitts 1b eingesetzt. Eine Temperaturdifferenz zwischen dem Kaltabschnitt 1b und dem Warmabschnitt 1a kann so vorteilhaft reduziert werden.

Die in Fig. 2 gezeigte Anordnung 100 kann in manchen Ausführungsformen als Einmalartikel ausgestaltet sein, in anderen Ausführungsformen jedoch auch als fester Hydraulikabschnitt z.B. einer medizinischen Behandlungsvorrichtung zum Einsatz kommen.

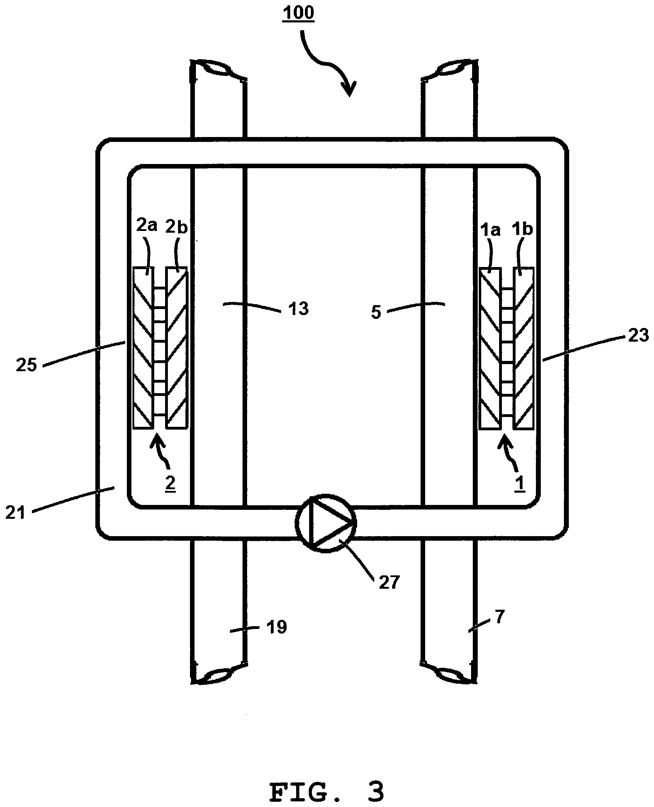

**Fig. 3** zeigt schematisch vereinfacht eine erfindungsgemäße Anordnung 100 in einer dritten Ausführungsform.

Fig. 3 veranschaulicht anstelle einer direkten Wärmeübertragung von der gebrauchten zur frischen Dialysierflüssigkeit, wie dies anhand von Fig. 2 gezeigt ist, eine Wärmeübertragung unter Einsatz eines Wärmeüberträgerfluids.

Das Wärmeüberträgerfluid ist in manchen erfindungsgemäßen Ausführungsformen eigens und/oder ausschließlich zum Übertragen von Wärmeenergie von einem Warmabschnitt einer Erwärmungseinrichtung auf einem Kaltabschnitt einer anderen Erwärmungseinrichtung vorgesehen.

Die erfindungsgemäße Anordnung 100 weist einen Wärmeüberträgerfluidkreislauf 21 mit einem Wärmeüberträgerfluid, zum Beispiel ein Öl, Wasser oder dergleichen auf.

Die erfindungsgemäße Anordnung 100 weist ferner eine zweite Erwärmungseinrichtung 2 auf. Die zweite Erwärmungseinrichtung 2 weist einen sich bei deren Betrieb erwärmenden zweiten Warmabschnitt 2a und einen sich bei deren Betrieb abkühlenden zweiten Kaltabschnitt 2b auf.

Der Wärmeüberträgerfluidkreislauf 21 weist einen ersten Wärmeüberträgerbereich 23 und einen zweiten Wärmeüberträgerbereich 25 auf.

Der Wärmeüberträgerfluidkreislauf 21 ist derart zwischen dem ersten Bereich 5 und dem zweiten Bereich 13 angeordnet, dass der erste Warmabschnitt 1a der ersten Erwärmungseinrichtung 1 in Wärmeaustauschbeziehung mit dem ersten Bereich 5 steht; der erste Kaltabschnitt 1b der ersten Erwärmungseinrichtung 1 steht mit dem ersten Wärmeüberträgerbereich 23 in Wärmeaustauschbeziehung, der zweite Warmabschnitt 2a der zweiten Erwärmungseinrichtung 2 mit dem zweiten Wärmeüberträgerbereich 25 in Wärmeaustauschbeziehung steht; und der zweite Kaltabschnitt 2b der zweiten Erwärmungseinrichtung 2 steht mit dem zweiten Bereich 13 in Wärmeaustauschbeziehung.

Bei dieser Anordnung 100 wird während des Betriebs zum Erwärmen des medizinischen Fluids im zweiten Bereich 13 zunächst mittels der zweiten Erwärmungseinrichtung 2 Wärme aus der gebrauchten Dialysierflüssigkeit des zweiten Bereichs 13 bzw. der Dialysierflüssigkeitsleitung 19 für gebrauchte Dialysierflüssigkeit auf das Wärmeüberträgerfluid im Wärmeüberträgerfluidkreislauf 21 übertragen. Hierzu wird zum Beispiel die der gebrauchten Dialysierflüssigkeit entzogene Wärme aus dem zweiten Bereich 13 auf den zweiten Kaltabschnitt 2b der zweiten Erwärmungseinrichtung 2 übertragen. Der zweite Kaltabschnitt 2b wird dadurch vorteilhaft erwärmt.

Durch Betreiben der zweiten Erwärmungseinrichtung 2 wird ferner elektrisch erzeugte Wärme aus dem zweiten Warmabschnitt 2a auf den zweiten Wärmeüberträgerbereich 25 übertragen.

Das dabei erwärmte Wärmeüberträgerfluid kann mittels einer geeigneten Fördereinrichtung, beispielsweise einer wie in Fig. 3 gezeigten Pumpe 27, zum ersten Kaltabschnitt 1b der ersten Erwärmungseinrichtung 1 gefördert werden.

An der ersten Erwärmungseinrichtung 1 erfolgt im ersten Wärmeüberträgerbereich 23 ein Wärmeübergang aus dem Wärmeüberträgerfluid auf den ersten Kaltabschnitt 1b der ersten Erwärmungseinrichtung 1. Der erste Kaltabschnitt 1b wird dabei vorteilhaft erwärmt.

Die frische Dialysierflüssigkeit der Dialysierflüssigkeitsleitung 7 für frische Dialysierflüssigkeit wird wiederum durch Betreiben der ersten Erwärmungseinrichtung 1 und Wärmeübergang vom ersten Warmabschnitt 1a auf den ersten Bereich 5 erwärmt.

Die in Fig. 3 beschriebene Anordnung kann in manchen Ausführungsformen den Vorteil bieten, frische und gebrauchte Dialysierflüssigkeit räumlich getrennt voneinander zu führen. Dennoch wird andernfalls verworfene Wärme der gebrauchten Dialysierflüssigkeit genutzt. Einer gegebenenfalls möglichen Kontaminationsgefahr kann so vorteilhaft vorgebeugt werden.

Die in Fig. 3 gezeigte Anordnung 100 kann als Einmalartikel oder als - fest installierter - Hydraulikabschnitt, vorgesehen zur mehrmaligen Verwendung, ausgestaltet sein.

Die vorliegende Erfindung wurde in Fig. 2 und 3 beispielhaft anhand von Ausführungsformen, in denen sowohl das medizinische Fluid als auch das zweite Fluid als Dialysierflüssigkeit vorliegen, beschrieben. Die Erfindung kann jedoch in gleicher Weise mit anderen Fluiden ausgeführt werden wie zu Fig. 1 beschrieben ist. Geeignete Beispiele schließen eine Anordnung ein, in denen anstelle der Dialysierflüssigkeitsströme die Wärme aus extrakorporalem Patientenblut entzogen wird, bevor dieses in einen (Blutbehandlung-)Filter geleitet wird, und diese Wärme dem Patientenblut zugeführt wird, bevor das Blut dem Patienten zurückgegeben wird.

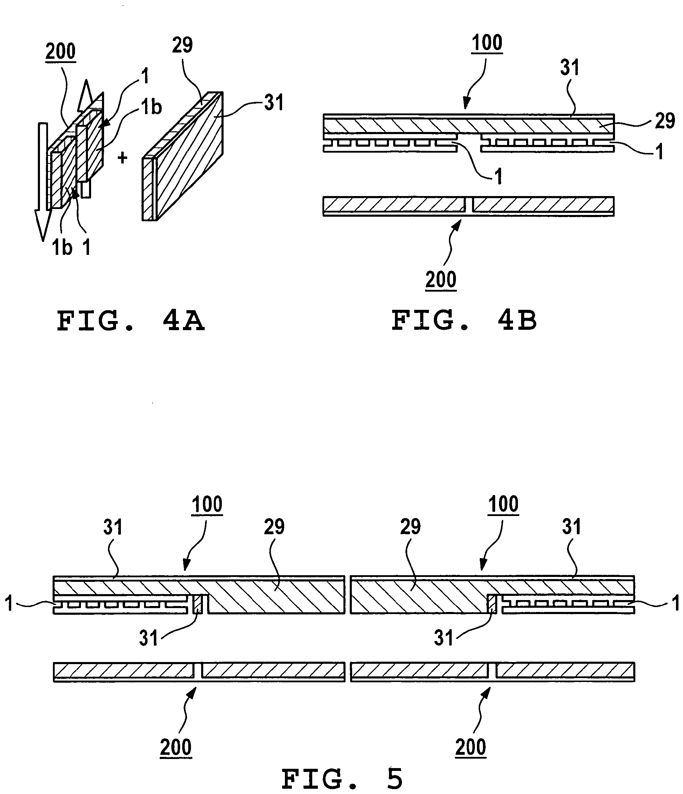

**Fig. 4A** zeigt in Perspektive schematisch vereinfacht eine erfindungsgemäße Anordnung 100 gemäß einer vierten Ausführungsform, in welcher eine Erwärmungseinrichtung 1, hier als Peltierheizung gezeigt, mit Wärmeaustauschfunktion an einem Einmalartikel 200 flächig angeordnet ist.

Am Kaltabschnitt 1b der Erwärmungseinrichtungen 1 ist vorgesehen, eine Wärmebrücke 29 anzuordnen, welche eine Isolation 31 aufweist.

Eine solche Ausführungsform, in der keine Heizflächen (Erwärmungseinrichtungen, Peltierelemente) aus der Maschinenfront einer Behandlungsvorrichtung ragen, kann verschiedene Vorteile bereitstellen. Solche Vorteile umfassen geringere Kosten für den Einmalartikel 200, eine bessere Handhabung, eine einfachere Konstruktion und dergleichen.

In den in Fig. 4A und 4B gezeigten Ausführungsformen sind zwei Erwärmungseinrichtungen 1 über eine Wärmebrücke 29 verbunden, um Wärme vom gebrauchten Dialysat an das kalte, frische Dialysat zu transportieren. Die Blockpfeile der Fig. 4A bezeichnen entsprechend den Strom von frischem und gebrauchtem Dialysat am Einmalartikel 200 vorbei.

**Fig. 4B** zeigt schematisch vereinfacht eine Anordnung 100 an einer Funktionseinrichtung, hier dem Einmalartikel 200.

Zur besseren Darstellbarkeit ist ein Spalt zwischen der Anordnung 100 und dem Einmalartikel 200 gezeigt.

**Fig. 5** zeigt schematisch vereinfacht eine erfindungsgemäße Anordnung 100 gemäß einer fünften Ausführungsform, in welcher während des Betriebs eine Erwärmungseinrichtung 1 wiederum flächig am Einmalartikel 200 angeordnet ist. Lediglich zur besseren Darstellbarkeit ist jedoch auch in Fig. 5 ein Spalt zwischen Einmalartikel 200 und Erwärmungseinrichtung 1 gezeigt.

Die Funktion eines flächigen aktiven Wärmetauschers lässt sich durch ein Peltierelement realisieren. In einer solchen Ausführungsform kann die Wärmebrücke 29 gleichzeitig oder gleichermaßen zur Kühl- oder Heizseite werden.

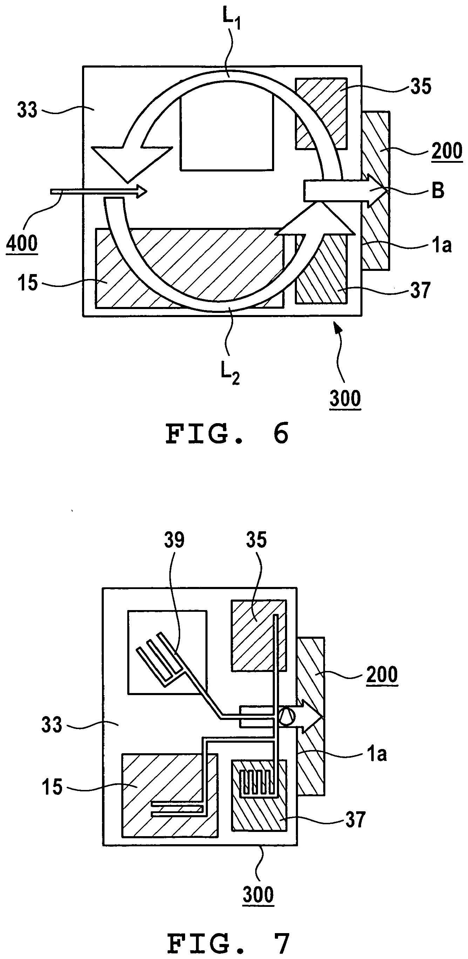

In den Fig. 6 bis 9 ist die Erwärmungseinrichtung jeweils Teil der Funktionseinrichtung. Sie ist in den Darstellungen der Fig. 6 bis 9 jeweils nicht zu sehen.

**Fig. 6** zeigt schematisch vereinfacht ein Wärmemanagement einer Behandlungsvorrichtung 300 mittels der erfindungsgemäßen Anordnung 100.

In der in Fig. 6 gezeigten Ausführungsform wird eine Erwärmung des Kaltabschnitts der Erwärmungseinrichtung mittels eines - insbesondere gezielt geführten - Luftstroms 400 (Lüfter) erreicht.

Die Erwärmungseinrichtung entzieht Wärme aus einem Maschineninnenraum 33 der Behandlungsvorrichtung 300 über den Warmabschnitt 1a der Erwärmungseinrichtung und überträgt diese auf den Einmalartikel 200, in welchem die Flüssigkeit erwärmt wird. Der Wärmetransport ist durch den Blockpfeil B zum Einmalartikel 200 hin veranschaulicht.

Durch die Zirkulation (dargestellt durch die gebogenen Pfeile L₁ und L₂) von Luft innerhalb der Behandlungsvorrichtung 300 kann der Luftaustausch zwischen vorhandener Abwärme der Elektronik 15, Sensorik 35 und/oder Aktorik 37 und der Erwärmungseinrichtung optimiert werden.

**Fig. 7** zeigt ein weiteres Wärmemanagement mittels der erfindungsgemäßen Anordnung 100 in einer Behandlungsvorrichtung 300.

In der in Fig. 7 gezeigten Ausführungsform wird eine Erwärmung des Kaltabschnitts der Erwärmungseinrichtung mittels einer - insbesondere gezielt geführten - Flüssigkeit erreicht.

Zum Ableiten der Abwärme der Elektronik 15, Sensorik 35 und/oder Aktorik 37 wird ein Fluidsystem 39 eingesetzt, welches Verteilerröhrchen, Wärmetauscher, Flüssigkeit und/oder (eine) Pumpe(n) aufweisen kann.

In einer solchen Ausführungsform kann die Behandlungsvorrichtung 300 vorteilhaft noch kompakter dimensioniert werden. Das Verwenden einer Flüssigkeit als Wärmeüberträger kann vorteilhaft die Wärmeübergänge und/oder die übertragene Wärmemenge optimieren.

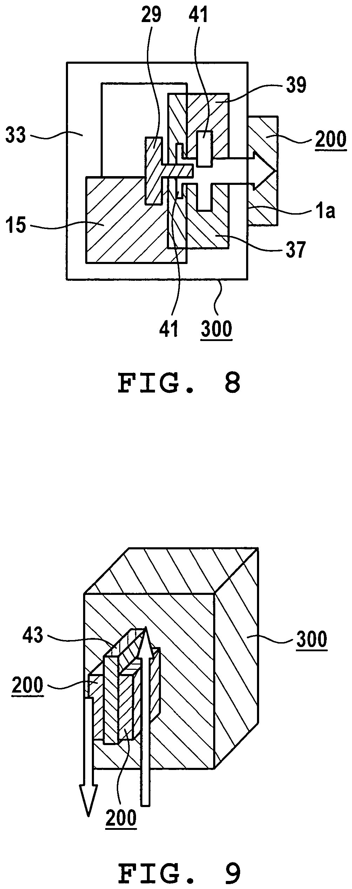

**Fig. 8** zeigt ein weiteres Wärmemanagement mittels der erfindungsgemäßen Anordnung 100 in oder an einer Behandlungsvorrichtung 300.

In der in Fig. 8 gezeigten Ausführungsform wird die Wärmeübertragung durch feste Materialien, wie eine Wärmebrücke 29, erreicht. In solchen Ausführungsformen kann auf die Verwendung eines Fluids zur Wärmeübertragung verzichtet werden. Letzteres kann allerdings ergänzend vorgesehen sein, ebenso eine Wärmeübertragung mittels - insbesondere gezielt geführter - Luft.

Die zu kühlenden Elemente werden durch mindestens einen Kühlblock 41 mit der Erwärmungseinrichtung verbunden. Für eine geringe Wärmeleistung kann eine Wärmeleitfolie für den Kühlblock 41 verwendet werden; bei hohen Wärmeleistungen können vorzugsweise Aluminium, Kupfer und/oder Keramiken wie Aluminiumnitrid (AlN) und dergleichen eingesetzt werden.

**Fig. 9** zeigt schematisch vereinfacht eine erfindungsgemäße Anordnung 100 an einer Behandlungsvorrichtung 300.

In der in Fig. 9 gezeigten Ausführungsform sind zwei Erwärmungseinrichtungen in jeweils einem Einmalartikel 200 rechts und links, bezogen auf die Darstellung der Fig. 9, von einer Kühlrippe 43 angeordnet.

Die Kühlrippe 43 ist von der Behandlungsvorrichtung 300 bzw. einem Inneren der Behandlungsvorrichtung 300 entkoppelt. Die Kühlrippe 43 ist an der Maschinenfront der Behandlungsvorrichtung 300 angeordnet, so dass sie von der Behandlungsvorrichtung 300 absteht bzw. aus dieser hervorragt.

An den Einmalartikeln 200 werden gebrauchtes Dialysat (Blockpfeil nach unten) und frisches Dialysat (Blockpfeil nach oben) vorbeigeleitet.

Das gebrauchte Dialysat gibt Wärme an den Kaltabschnitt der Erwärmungseinrichtung im linken Einmalartikel ab. Der Warmabschnitt dieser Erwärmungseinrichtung gibt Wärme an die Kühlrippe 43 ab. Die Kühlrippe 43 gibt Wärme an den Kaltabschnitt der Erwärmungseinrichtung im rechten Eimalartikel für das frische Dialysat ab. Der Warmabschnitt dieser Erwärmungseinrichtung wird dazu verwendet, das frische Dialysat zu erwärmen, so dass am Blut warmes frisches Dialysat vorbeigeleitet wird.

Die in Fig. 9 gezeigte Ausführungsform kann den Vorteil bereitstellen, dass die Kühlrippe 43 unabhängig vom Maschineninnenraum der Behandlungsvorrichtung 300 ist.

**Bezugszeichenliste**

| **Bezugszeichen** | **Beschreibung** |
|---|---|
| 100 | Anordnung |
| 200 | Einmalartikel |
| 300 | Behandlungsvorrichtung |
| 400 | Luftstrom |
| 1 | erste Erwärmungseinrichtung |
| 1a | erster Warmabschnitt |
| 1b | erster Kaltabschnitt |
| 2 | zweite Erwärmungseinrichtung |
| 2a | zweiter Warmabschnitt |
| 2b | zweiter Kaltabschnitt |
| 3 | Stromkreis |
| 5 | erster Bereich |
| 7 | Dialysierflüssigkeitsleitung (frische Dialysierflüssigkeit) |
| 9 | Fördereinrichtung |
| 11 | Kühlkörper |
| 13 | zweiter Bereich |
| 15 | elektronische Bauteile |
| 17 | Lüfter |
| 18 | Mäanderstruktur |
| 19 | Dialysierflüssigkeitsleitung (gebrauchte Dialysierflüssigkeit) |
| 21 | Wärmeüberträgerfluidkreislauf |
| 23 | erster Wärmeüberträgerbereich |
| 25 | zweiter Wärmeüberträgerbereich |
| 27 | Pumpe |
| 29 | Wärmebrücke |
| 31 | Isolation |
| 33 | Maschineninnenraum |
| 35 | Sensorik |
| 37 | Aktorik |
| 39 | Fluidsystem |
| 41 | Kühlblock |
| 43 | Kühlrippe |
| B | Blockpfeil zur Veranschaulichung des Wärmetransports |
| L₁, L2 | Zirkulation |

## Patentansprüche

1. Anordnung (100) zum Erwärmen einer Dialysierflüssigkeit im Rahmen einer extrakorporalen Blutbehandlung, aufweisend:
- wenigstens eine elektrisch betriebene Erwärmungseinrichtung (1) mit wenigstens einem sich bei deren Betrieb erwärmenden Warmabschnitt (1a) und wenigstens einem sich bei deren Betrieb abkühlenden Kaltabschnitt (1b);
- einen ersten Bereich (5) zum Aufnehmen der zu erwärmenden Dialysierflüssigkeit, wobei der erste Bereich (5) in Wärmeaustauschbeziehung mit dem Warmabschnitt der Erwärmungseinrichtung (1) steht; und
- einen zweiten Bereich (13) zum Aufnehmen einer gebrauchten Dialysierflüssigkeit, welcher in Wärmeaustauschbeziehung mit dem Kaltabschnitt (1b) der Erwärmungseinrichtung steht,
wobei der Bereich zum Aufnehmen der zu erwärmenden Dialysierflüssigkeit (5) und der Bereich zum Aufnehmen der gebrauchten Dialysierflüssigkeit (13) Teile desselben Kreislaufs sind.

2. Anordnung (100) zum Erwärmen einer Dialysierflüssigkeit im Rahmen einer extrakorporalen Blutbehandlung, aufweisend:
- wenigstens eine elektrisch betriebene erste Erwärmungseinrichtung (1) mit wenigstens einem sich bei deren Betrieb erwärmenden ersten Warmabschnitt (1a) und wenigstens einem sich bei deren Betrieb abkühlenden ersten Kaltabschnitt (1b);
- wenigstens eine elektrisch betriebene zweite Erwärmungseinrichtung (2) mit wenigstens einem sich bei deren Betrieb erwärmenden zweiten Warmabschnitt (2a) und wenigstens einem sich bei deren Betrieb abkühlenden zweiten Kaltabschnitt (2b);
- einen ersten Bereich (5) zum Aufnehmen der zu erwärmenden Dialysierflüssigkeit, wobei der erste Bereich (5) in Wärmeaustauschbeziehung mit dem ersten Warmabschnitt (1a) der ersten Erwärmungseinrichtung (1) steht; und
- einen zweiten Bereich (13) zum Aufnehmen einer gebrauchten Dialysierflüssigkeit, wobei der zweite Bereich (13) in Wärmeaustauschbeziehung mit dem zweiten Kaltabschnitt (2b) der zweiten Erwärmungseinrichtung (2) ist, und
- wobei der Bereich zum Aufnehmen der zu erwärmenden Dialysierflüssigkeit (5) und der Bereich zum Aufnehmen der gebrauchten Dialysierflüssigkeit (13) Teile desselben Kreislaufs sind.

3. Anordnung (100) nach Anspruch 1 oder 2, vorgesehen zum Erwärmen der Dialysierflüssigkeit in einem ersten Zeitabschnitt und vorgesehen zum Abkühlen der Dialysierflüssigkeit in einem zweiten Zeitabschnitt, welcher vom ersten Zeitabschnitt verschieden ist.

4. Anordnung (100) nach Anspruch 3, mit einer Einrichtung zum zumindest vorübergehenden Veranlassen einer Abkühlung des Dialysierflüssigkeit mittels des ersten Warmabschnitts (1a), anstelle von dessen Erwärmung mittels des ersten Warmabschnitts (1a) durch Umkehr einer Stromzufuhr für die elektrisch betriebene erste Erwärmungseinrichtung (1).

5. Anordnung (100) nach einem der vorangegangenen Ansprüche, wobei die erste Erwärmungseinrichtung (1) wenigstens ein Peltier-Element ist oder aufweist.

6. Anordnung (100) nach einem der vorangegangenen Ansprüche, wobei der zweite Bereich (13) mit dem ersten Kaltabschnitt (1b) der ersten Erwärmungseinrichtung (1) oder mit dem zweiten Kaltabschnitt (2b) der zweiten Erwärmungseinrichtung (2) zum Erwärmen des ersten Kaltabschnitts (1b) oder des zweiten Kaltabschnitts (2b) verbunden ist.

7. Anordnung (100) nach Anspruch 2 oder 6, ferner aufweisend:
- wenigstens einen Wärmeüberträgerfluidkreislauf (21) mit einem ersten Wärmeüberträgerbereich (23) und einem zweiten Wärmeüberträgerbereich (25), jeweils vorgesehen zum Aufnehmen eines Wärmeüberträgerfluids,
wobei der Wärmeüberträgerfluidkreislauf (21) derart zwischen dem ersten Bereich (5) und dem zweiten Bereich (13) angeordnet ist, dass der erste Warmabschnitt (1a) der ersten Erwärmungseinrichtung (1) in Wärmeaustauschbeziehung mit dem ersten Bereich (5) steht, der erste Kaltabschnitt (1b) der ersten Erwärmungseinrichtung (1) mit dem ersten Wärmeüberträgerbereich (23) in Wärmeaustauschbeziehung steht, der zweite Warmabschnitt (2a) der zweiten Erwärmungseinrichtung (2) mit dem zweiten Wärmeüberträgerbereich (25) in Wärmeaustauschbeziehung steht und der zweite Kaltabschnitt (2b) der zweiten Erwärmungseinrichtung (2) mit dem zweiten Bereich (13) in Wärmeaustauschbeziehung steht.

8. Anordnung (100) nach einem der vorangegangenen Ansprüche, ausgestaltet als wiederverwendbare Einheit.

9. Anordnung (100) nach einem der Ansprüche 1 bis 8, ausgestaltet als Einmalteil.

10. Medizinische Funktionseinrichtung mit wenigstens einer Anordnung (100) gemäß einem der Ansprüche 1 bis 9.

11. Medizinische Behandlungsvorrichtung mit wenigstens einer Anordnung (100) gemäß einem der Ansprüche 1 bis 9 oder mit einer medizinischen Funktionseinrichtung gemäß Anspruch 10.

12. Verfahren zum Temperieren einer Dialysierflüssigkeit mit dem Schritt:
- Verwenden einer Anordnung (100) gemäß einem der Ansprüche 1 bis 9.

13. Verfahren nach Anspruch 12 zum Erwärmen einer Dialysierflüssigkeit mit den Schritten:
- Einbringen einer Dialysierflüssigkeit in den ersten Bereich (5);
- Erwärmen der Dialysierflüssigkeit im ersten Bereich (5) mittels der ersten Erwärmungseinrichtung (1) durch Übertragen von Wärme vom ersten Warmabschnitt (1a) der ersten Erwärmungseinrichtung (1) auf den ersten Bereich (5);
- Gezieltes Führen oder Leiten der Dialysierflüssigkeit durch den ersten und/oder den zweiten Bereich (5; 13), und
- Erwärmen des Kaltabschnitts (1b; 2b) der ersten oder der zweiten Erwärmungseinrichtung (1; 2) durch Übertragen von Wärme der gebrauchten Dialysierflüssigkeit des zweiten Bereichs (13) auf den Kaltabschnitt (1b; 2b) der ersten oder der zweiten Erwärmungseinrichtung (1; 2).

14. Verfahren nach Anspruch 12 zum Erwärmen einer Dialysierflüssigkeit mit den Schritten:
- Verwenden einer Anordnung (100) insbesondere gemäß Anspruch 7;
- Einbringen einer Dialysierflüssigkeit in den ersten Bereich (5);
- Erwärmen der Dialysierflüssigkeit im ersten Bereich (5) mittels der ersten Erwärmungseinrichtung (1) durch Übertragen von Wärme vom ersten Warmabschnitt (1a) der ersten Erwärmungseinrichtung (1) auf den ersten Bereich (5);
- Erwärmen des zweiten Kaltabschnitts (2b) der zweiten Erwärmungseinrichtung (2) durch Übertragen von Wärme der gebrauchten Dialysierflüssigkeit des zweiten Bereichs (13) auf den zweiten Kaltabschnitt (2b) der zweiten Erwärmungseinrichtung (2);
- Verwenden eines Wärmeüberträgerfluids in einem Wärmeüberträgerfluidkreislauf (21) mit einem ersten Wärmeüberträgerbereich (23) und einen zweiten Wärmeüberträgerbereich (25), wobei der Wärmeüberträgerfluidkreislauf (21) derart zwischen dem ersten Bereich (5) und dem zweiten Bereich (13) angeordnet ist, dass der erste Warmabschnitt (1a) der ersten Erwärmungseinrichtung (1) in Wärmeaustauschbeziehung mit dem ersten Bereich (5) steht, der erste Kaltabschnitt (1b) der ersten Erwärmungseinrichtung (1) mit dem ersten Wärmeüberträgerbereich (23) in Wärmeaustauschbeziehung steht, der zweite Warmabschnitt (2a) der zweiten Erwärmungseinrichtung (2) mit dem zweiten Wärmeüberträgerbereich (25) in Wärmeaustauschbeziehung steht und der zweite Kaltabschnitt (2b) der zweiten Erwärmungseinrichtung (2) mit dem zweiten Bereich (13) in Wärmeaustauschbeziehung steht;
- Erwärmen des Wärmeüberträgerfluids im zweiten Wärmeüberträgerbereich (25) mittels der zweiten Erwärmungseinrichtung (2) durch Übertragen von Wärme vom zweiten Warmabschnitt (2a) der zweiten Erwärmungseinrichtung (2) auf den zweiten Wärmeüberträgerbereich (25); und
- Übertragen von Wärme des Wärmeüberträgerfluids des ersten Wärmeüberträgerbereichs (23) auf den ersten Kaltabschnitt (1b) der ersten Erwärmungseinrichtung (1).

15. Verfahren nach Anspruch 12zum Erwärmen einer Dialysierflüssigkeit in einem ersten Zeitabschnitt und zum Abkühlen der Dialysierflüssigkeit in einem zweiten Zeitabschnitt, welcher vom ersten Zeitabschnitt verschieden ist, mit den Schritten:
- Verwenden einer Anordnung (100) insbesondere gemäß Anspruch 3;
- Einbringen einer Dialysierflüssigkeit in den ersten Bereich (5);
- Erwärmen der Dialysierflüssigkeit im ersten Bereich (5) mittels der ersten Erwärmungseinrichtung (1) durch Übertragen von Wärme vom ersten Warmabschnitt (1a) der ersten Erwärmungseinrichtung (1) auf den ersten Bereich (5); und
- Verändern einer Stromzufuhr der elektrisch betriebenen ersten Erwärmungseinrichtung (1) und/oder der elektrisch betriebenen zweiten Erwärmungseinrichtung (2) derart, dass die Dialysierflüssigkeit im ersten Bereich (5) mittels der ersten Erwärmungseinrichtung (1) durch Übertragen von Kälte vom ersten Warmabschnitt (1a) der ersten Erwärmungseinrichtung (1) auf den ersten Bereich (5) abgekühlt wird.

## Claims

1. An arrangement (100) for heating a dialysis liquid during an extracorporeal blood treatment, comprising:
- at least one electrically operated heating device (1) comprising at least one warm section (1a) heating during operation of the heating device, and at least one cold section (1b) cooling during operation of the heating device;
- a first area (5) for receiving the dialysis liquid to be heated, wherein the first area (5) is in heat exchange relation with the warm section of the heating device (1); and
- a second area (13) for receiving a used dialysis liquid that is in heat exchange relation with the cold section (1b) of the heating device,
wherein the area (5) for receiving the dialysis liquid to be heated and the area (13) for receiving the used dialysis liquid are part of the same circuit.

2. The arrangement (100) for heating a dialysis liquid during an extracorporeal blood treatment, comprising:
- at least one electrically operated first heating device (1) comprising at least one first warm section (1a) heating during operation of the first heating device and at least one first cold section (1b) cooling during operation of the first heating device;
- at least one electrically operated second heating device (2) comprising at least one second warm section (2a) heating during operation of the second heating device and at least one second cold section (2b) cooling during operation of the second heating device;
- a first area (5) for receiving the dialysis liquid to be heated, wherein the first area (5) is in heat exchange relation with the first warm section (1a) of the first heating device (1); and
- a second area (13) for receiving a used dialysis liquid, wherein the second area (13) is in heat exchange relation with the second cold section (2b) of the second heating device (2), and
- wherein the area (5) for receiving the dialysis liquid to be heated and the area (13) for receiving the used dialysis liquid are part of the same circuit.

3. The arrangement (100) according to claim 1 or 2, provided or intended for heating the dialysis liquid in a first time interval and provided or intended for cooling the dialysis liquid in a second time interval differing from the first time interval.

4. The arrangement (100) according to claim 3, comprising a device for at least temporary prompting the cooling of the dialysis liquid by means of the first warm section (1a) instead of heating the dialysis liquid by means of the first warm section (1a) by reversing a current supply for the electrically operated first heating device (1).

5. The arrangement (100) according to anyone of the preceding claims, wherein the first heating device (1) is or comprises at least one Peltier element.

6. The arrangement (100) according to anyone of the preceding claims, wherein the second area (13) is connected with the first cold section (1b) of the first heating device (1) or with the second cold section (2b) of the second heating device (2) for heating the first cold section (1b) or the second cold section (2b).

7. The arrangement (100) according to claim 2 or 6, further comprising:
- at least one heat transfer fluid circuit (21) comprising a first heat transfer area (23) and a second heat transfer area (25), each provided or intended for receiving a heat transfer fluid,
wherein the heat transfer fluid circuit (21) is arranged between the first area (5) and the second area (13) such that the first warm section (1a) of the first heating device (1) is in heat exchange relation with the first area (5), the first cold section (1b) of the first heating device (1) is in heat exchange relation with the first heat transfer area (23), the second warm section (2a) of the second heating device (2) is in heat exchange relation with the second heat transfer area (25) and the second cold section (2b) of the second heating device (2) is in heat exchange relation with the second area (13).

8. The arrangement (100) according to anyone of the preceding claims, designed or embodied as a reusable unit.

9. The arrangement (100) according to anyone of claims 1 to 8, designed or embodied as a single-use article.

10. A medical functional means comprising at least one arrangement (100) according to anyone of claims 1 to 9.

11. A medical treatment apparatus comprising at least one arrangement (100) according to anyone of claims 1 to 9 or comprising a medical functional means according to claim 10.

12. A method for tempering a dialysis liquid, comprising the step of:
- using an arrangement (100) according to anyone of claims 1 to 9.

13. The method according to claim 12 for heating a dialysis liquid, comprising the steps of:
- introducing a dialysis liquid into the first area (5);
- heating the dialysis liquid in the first area (5) by means of the first heating device (1) by transferring heat from the first warm section (1a) of the first heating device (1) onto the first area (5);
- conducting or directing the dialysis liquid through the first and/or the second area (5; 13) in a targeted manner, and
- heating the cold section (1b; 2b) of the first or of the second heating device (1; 2) by transferring heat of the used dialysis liquid of the second area (13) onto the cold section (1b; 2b) of the first or of the second heating device (1; 2).

14. The method according to claim 12 for heating a dialysis liquid, comprising the steps of:
- using an arrangement (100), in particular according to claim 7;
- introducing a dialysis liquid into the first area (5);
- heating the dialysis liquid in the first area (5) by means of the first heating device (1) by transferring heat from the first warm section (1a) of the first heating device (1) onto the first area (5);
- heating the second cold section (2b) of the second heating device (2) by transferring heat of the used dialysis liquid of the second area (13) onto the second cold section (2b) of the second heating device (2);
- using a heat transfer fluid within a heat transfer fluid circuit (21) comprising a first heat transfer area (23) and a second heat transfer area (25), wherein the heat transfer fluid circuit (21) is arranged between the first area (5) and the second area (13) such that the first warm section (1a) of the first heating device (1) is in heat exchange relation with the first area (5), the first cold section (1b) of the first heating device (1) is in heat exchange relation with the first heat transfer area (23), the second warm section (2a) of the second heating device (2) is in heat exchange relation with the second heat transfer area (25) and the second cold section (2b) of the second heating device (2) is in heat exchange relation with the second area (13);
- heating the heat transfer fluid in the second heat transfer area (25) by means of the second heating device (2) by transferring heat from the second warm section (2a) of the second heating device (2) onto the second heat transfer area (25); and
- transferring heat of the heat transfer fluid of the first heat transfer area (23) onto the first cold section (1b) of the first heating device (1).

15. The method according to claim 12 for heating a dialysis liquid in a first time interval and for cooling the dialysis liquid in a second time interval differing from the first time interval, comprising the steps of:
- using an arrangement (100), in particular according to claim 3;
- introducing a dialysis liquid into the first area (5);
- heating the dialysis liquid in the first area (5) by means of the first heating device (1) by transferring heat from the first warm section (1a) of the first heating device (1) onto the first area (5); and
- changing a power supply of the electrically operated first heating device (1) and/or of the electrically operated second heating device (2) such that the dialysis liquid in the first area (5) is cooled by means of the first heating device (1) by transferring cold from the first warm section (1a) of the first heating device (1) onto the first area (5).

## Revendications

1. Un arrangement (100) destiné à chauffer un liquide de dialyse pendant un traitement du sang extracorporel, comprenant:
- au moins un dispositif de chauffage (1) actionné électriquement avec au moins une partie chaude (1a) chauffant lors de l'opération du dispositif de chauffage, et au moins une partie froide (1b) refroidissant lors de l'opération du dispositif de chauffage;
- une première région (5) destinée à recevoir le liquide de dialyse devant être chauffé, où la première région (5) est en relation d'échange thermique avec la partie chaude du dispositif de chauffage (1), et
- une deuxième région (13) destinée à recevoir un liquide de dialyse usagé disposée en relation d'échange thermique avec la partie froide (1b) du dispositif de chauffage,
dans lequel la région (5) destinée à recevoir le liquide de dialyse devant être chauffé et la région (13) destinée à recevoir le liquide de dialyse usagé font partie du même circuit.

2. Un arrangement (100) destiné à chauffer un liquide de dialyse pendant un traitement du sang extracorporel comprenant:
- au moins un premier dispositif de chauffage (1) actionné électriquement avec au moins une première partie chaude (1a) chauffant lors de l'opération du premier dispositif de chauffage, et au moins une première partie froide (1b) refroidissant lors de l'opération du premier dispositif de chauffage;
- au moins un deuxième dispositif de chauffage (2) actionné électriquement avec au moins une deuxième partie chaude (2a) chauffant lors de l'opération du deuxième dispositif de chauffage, et au moins une deuxième partie froide (2b) refroidissant lors de l'opération du deuxième dispositif de chauffage;
- une première région (5) destinée à recevoir le liquide de dialyse devant être chauffé, où la première région (5) est en relation d'échange thermique avec la première partie chaude (1a) du premier dispositif de chauffage (1), et
- une deuxième région (13) destinée à recevoir un liquide de dialyse usagé, où la deuxième région (13) est en relation d'échange thermique avec la deuxième partie froide (2b) du deuxième dispositif de chauffage (2), et
dans lequel la région (5) destinée à recevoir le liquide de dialyse devant être chauffé et la région (13) destinée à recevoir le liquide de dialyse usagé font partie du même circuit.

3. L'arrangement (100) selon la revendication 1 ou 2, destiné à ou prévu pour chauffer le liquide de dialyse pendant un premier intervalle de temps et destiné à ou prévu pour refroidir le liquide de dialyse pendant un deuxième intervalle de temps différent du premier intervalle de temps.

4. L'arrangement (100) selon la revendication 3, comprenant un dispositif destiné à entrainer, tout au moins temporairement, un refroidissement du liquide de dialyse au moyen de la première partie chaude (1a) au lieu de chauffer le liquide de dialyse au moyen de la première partie chaude (1a) en inversant l'alimentation en courant du premier dispositif de chauffage (1) actionné électriquement (1).

5. L'arrangement (100) selon l'une quelconque des revendications précédentes, dans lequel le premier dispositif de chauffage (1) est ou comprend au moins un élément à effet Peltier.

6. L'arrangement (100) selon l'une quelconque des revendications précédentes, dans lequel la deuxième région (13) est connectée avec la première partie froide (1b) du premier dispositif de chauffage (1) ou avec la deuxième partie froide (2b) du deuxième moyen de chauffage (2) pour chauffer la première partie froide (1b) ou la deuxième partie froide (2b).

7. L'arrangement (100) selon la revendication 2 ou 6, comprenant de plus:
- au moins un circuit fluidique caloporteur (21) avec au moins une première région caloporteuse (23) et une deuxième région caloporteuse (25) chacune étant destinée ou prévue pour recevoir un fluide caloporteur,
- dans lequel le circuit fluidique caloporteur (21) est disposé entre la première région (5) et la deuxième région (13), de façon à ce que la première partie chaude (1a) du premier dispositif de chauffage (1) soit en relation d'échange thermique avec la première région (5), la première partie froide (1b) du premier dispositif de chauffage (1) soit en relation d'échange thermique avec la première région caloporteuse (23), la deuxième partie chaude (2a) du deuxième dispositif de chauffage (2) soit en relation d'échange thermique avec la deuxième région caloporteuse (25), et la deuxième partie froide (2b) du deuxième dispositif de chauffage (2) soit en relation d'échange thermique avec la deuxième région (13).

8. L'arrangement (100) selon l'une quelconque des revendications précédentes, configuré ou conçu comme une unité réutilisable.

9. L'arrangement (100) selon l'une quelconque des revendications 1 à 8, configuré ou conçu comme article à usage unique.

10. Un dispositif médical fonctionnel avec au moins un arrangement (100) selon l'une des revendications 1 à 9.

11. Un appareil de traitement médical avec au moins un arrangement (100) selon l'une quelconque des revendications 1 à 9 ou avec un dispositif médical fonctionnel selon la revendication 10.

12. Un procédé destiné à tempérer un liquide de dialyse comprenant l'étape consistant à:
- utiliser au moins un arrangement (100) selon l'une quelconque des revendications 1 à 9.

13. Le procédé selon la revendication 12 destiné à chauffer un liquide de dialyse comprenant les étapes consistant à:
- introduire un liquide de dialyse dans la première région (5);
- chauffer le liquide de dialyse dans la première région (5) au moyen du premier dispositif de chauffage (1) en transférant de la chaleur à partir de la première partie chaude (1a) du premier dispositif de chauffage (1) vers la première région (5);
- diriger ou conduire le fluide de dialyse au travers de la première et/ou de la deuxième région (5 ; 13) de façon ciblée; et
- chauffer la partie froide (1b ; 2b) du premier ou du deuxième dispositif de chauffage (1 ; 2) en transférant de la chaleur du liquide de dialyse usagé de la deuxième région (13) à la partie froide (1b ; 2b) du premier ou du deuxième dispositif de chauffage (1 ; 2).

14. Le procédé selon la revendication 12 destiné à chauffer un liquide de dialyse comprenant les étapes consistant à:
- utiliser un arrangement (100), en particulier selon la revendication 7;
- introduire un liquide de dialyse dans la première région (5);
- chauffer le liquide de dialyse dans la première région (5) au moyen du premier dispositif de chauffage (1) en transférant de la chaleur à partir de la première partie chaude (1a) du premier dispositif de chauffage (1) vers la première région (5);
- chauffer la deuxième partie froide (2b) du deuxième dispositif de chauffage (2) en transférant de la chaleur du liquide de dialyse usagé de la deuxième région (13) à la deuxième partie froide (2b) du deuxième dispositif de chauffage (2);
- utiliser un fluide caloporteur dans un circuit fluidique caloporteur (21) ayant une première région caloporteuse (23) et une deuxième région caloporteuse (25), où le circuit fluidique caloporteur (21) est disposé entre la première région (5) et la deuxième région (13), de façon à ce que la première partie chaude (1a) du premier dispositif de chauffage (1) soit en relation d'échange thermique avec la première région (5), la première partie froide (1b) du premier dispositif de chauffage (1) soit en relation d'échange thermique avec la première région caloporteuse (23), la deuxième partie chaude (2a) du deuxième dispositif de chauffage (2) soit en relation d'échange thermique avec la deuxième région caloporteuse (25), et la deuxième partie froide (2b) du deuxième dispositif de chauffage (2) soit en relation d'échange thermique avec la deuxième région (13);
- chauffer le fluide caloporteur dans la deuxième région caloporteuse (25) au moyen du deuxième dispositif de chauffage (2) en transférant de la chaleur à partir de la deuxième partie chaude (2a) du deuxième dispositif de chauffage (2) vers la deuxième région caloporteuse (25); et
- transférer de la chaleur du fluide caloporteur de la première région caloporteuse (23) vers la première partie froide (1b) du premier dispositif de chauffage (1).

15. Le procédé selon la revendication 12 destiné à chauffer un liquide de dialyse pendant un premier intervalle de temps et destiné à refroidir le liquide de dialyse pendant un deuxième intervalle de temps différent du premier intervalle de temps, comprenant les étapes consistant à:
- utiliser un arrangement (100), en particulier selon la revendication 3;
- introduire un liquide de dialyse dans la première région (5);
- chauffer le liquide de dialyse dans la première région (5) au moyen du premier dispositif de chauffage (1) en transférant de la chaleur à partir de la première partie chaude (1a) du premier dispositif de chauffage (1) vers la première région (5); et
- modifier une alimentation en courant du premier dispositif de chauffage (1) actionné électriquement et / ou du deuxième dispositif de chauffage (2) actionné électriquement de façon à ce que le liquide de dialyse dans la première région (5) soit refroidi au moyen du premier dispositif de chauffage (1) par le transfert de froid à partir de la première partie chaude (1a) du premier dispositif de chauffage (1) vers la première région (5).
